Europäisches Patentamt

**European Patent Office**

**Office européen des brevets**

(11) Publication number: **0 116 283**
**A1**

(12) **EUROPEAN PATENT APPLICATION**

(21) Application number: **84100181.1**

(22) Date of filing: **10.01.84**

(51) Int. Cl.³: **C 23 C 3/00**
**C 07 C 91/00**

(30) Priority: **17.01.83 US 458196**

(43) Date of publication of application:
**22.08.84 Bulletin 84/34**

(84) Designated Contracting States:
**IT NL SE**

(71) Applicant: **KOLLMORGEN TECHNOLOGIES**
**CORPORATION**
**66 Gatehouse Road**
**Stamford Connecticut 06902(US)**

(72) Inventor: **Paunovic, Milan**
**78 Shadyside Avenue**
**Port Washington, NY 11050(US)**

(72) Inventor: **Nuzzi, Francis J.**
**308 Locust Avenue**
**Freeprt NY 11520(US)**

(74) Representative: **Königseder, Claudia**
**Zugspitzstrasse 65**
**D-8104 Grainau(DE)**

(54) Treatment of EDTP for use in electroless copper baths.

(57) The improvement in electroless copper plating baths for the deposition of copper onto a substrate wherein ethylenedinitrilotetra-2-propanol (EDTP) is used as complexer. The low boiling contaminants of the commercially available EDTP are removed by a process which avoids the formation of oxidation by-products. The thus treated EDTP renders good and consistent results with respect to deposition rate and copper quality of the deposit.

EP 0 116 283 A1

# TREATMENT OF EDTP FOR USE IN ELECTROLESS COPPER BATHS

In 1956 Atkinson, US patent 3 119 709, has suggested the use of EDTP (ethylenedinitrilotetra-2-propanol) as an effective chelating agent for copper in electroless copper plating baths.

Since, EDTP has been suggested for use in electroless copper plating baths because of the increased plating rate achieveable with EDTP as a complexant compared to EDTA (ethylenediaminetetraacetic acid) or Rochelle salt generally used for such purposes.

Commercially available EDTP products to be used for production scale purposes were, however, found to produce lot-to-lot dependent results with the majority of EDTP production batches causing copper deposits of completely unacceptable structure and quality and, thus, unpredictable and often extremely high reject rates.

EDTP is widely used as a polyol in the manufacture of urethane polymers and, therefore, the manufacturing quality control is mainly concerned with color and moisture content. No control is exercised with respect to surface active and other chelating impurities formed in the course of the EDTP production process or otherwise introduced into the product and detrimental to the quality of electrolessly formed copper deposits and to the EDTP containing electroless copper plating baths.

Up to this time, there has been no reliable way to make EDTP of a quality suitable for electroless depositions solutions. Although EDTP was known and used, adequate quality EDTP for this purpose was not available. Expensive selection methods were employed to pick the least contaminated of commercially available EDTP lots. Even attempts to select the usable product from a wide range of available batches were unsuccessful. Thus the otherwise superior electroless copper baths could not find wide acceptance due to quality failure of EDTP.

It is expected that these basic deficiencies would become more pronounced in the future in view of the trend to use cyanide-free baths to achieve high copper deposition rates where the copper quality is affected by purity to a greater extent.

It is the objective of the present invention to provide an EDTP product for use in electroless copper plating baths which overcomes the deficiencies and shortcomings of the previously used and commercially available EDTP.

More particularly, it is highly desirable to provide a product which renders consistent results in electroless copper plating baths and is free of lot-to-lot variations and which consistently produces copper deposits which are coherent and ductile.

The production of pure EDTP in lab scale processes is prior art (compare Lunsted et al US patent 2 697 118). In the process suggested therein, EDTP is manufactured by reacting propylene oxide and ethylene diamine. Ethylene diamine is introduced into the reactor and heated to 60°C. Four moles of propylene oxide per mole of ethylene diamine are introduced into the reactor to a pressure of 70 kPa. The reaction mixture is continuously stirred and cooled to maintain the temperature between 60 and 100°C. After an hour or more, the pressure stabilizes signaling the reaction is complete. The EDTP is then vacuum stripped to remove water. Such product as achieved by the process described in US patent 2 697 118 would certainly fulfil the conditions outlined above, but would be far too expensive for production purposes on an extensive scale.

The applicants of the present invention have found that the above mentioned problems were caused by surprisingly small amounts of contaminants in the EDTP, and that such contaminants are characterized by higher vapor pressure and lower boiling points that EDTP. Surprisingly, removal of these small amounts of low boilers does produce EDTP of uniform quality for electroless plating baths and does produce good electroless copper deposits.

In accordance with the present invention there is provided a comparatively inexpensive and simple method for use in an electroless copper plating bath for the deposition of copper onto a substrate comprising at least a water soluble copper salt, formaldehyde, hydroxide to adjust pH, a wetting agent and EDTP, ethylenedinitrilotetra-2-propanol, as complexer for the copper ions, characterized in that the low boiling impurities contained in commercially available EDTP are removed by a process during which no oxidation by-products are formed.

It has been found that EDTP which contains less than 0,2 % of the above mentioned low boiling impurities used as a complexer in an electroless copper bath is characterized by the ability to consistently provide, in a test solution, a copper plating rate of between 4,8 and 8,8 µm per hour at a temperature of between 24 and 26°C and a copper deposit on a stainless steel strip which is smooth, non-flakey, unstressed and bright in color.

As described hereinafter and in the Examples, the impurities can be removed in accordance with the present invention by either chromatographic means or by pre-electrolysis or by stripping.

The EDTP containing electroless copper baths disclosed herein are highly effective as they allow the production of consistently uniform, coherent and ductile copper deposits. By using the EDTP product defined herein with attendant bath characteristics, the lot-to-lot variation problem assciated with previously used EDTP-containing baths is alleviated. EDTP is manufactured by condensing propylene oxide with ethylene diamine. In this reaction each amino hydrogen atom present in the diamine reacts with one molecule of propylene oxide. If reaction components are not present in the proper ratio and if the reaction is not properly controlled an incompletely hydroxypropylated derivative of ethylene diamine can result. Also, under different conditions, propylene oxide can further react with the active hydrogen of EDTP producing higher hydroxypropylated derivatives of ethylene diamine. Thus, most frequent impurities in a manufactured EDTP are (1) unreacted starting compounds, ethylene diamine and propylene oxide; (2) incompletely hydroxypropylated derivatives of ethylene diamine; (3) higher hydroxypropylated derivatives which are results of reaction of propylene oxide with EDTP; (4) decomposition products of EDTP produced during manufacture if the reaction mixture is exposed to air and higher temperatures; and (5) impurities such as acetone, propionaldehyde, ethylamines and ethylene diamine dimers and trimers contained in the starting raw materials. Inconsistencies in the process control and starting raw materials result in inconsistencies in composition of EDTP and in inconsistencies in physical properties of electrolessly deposited copper.

An alternative manufacturing procedure for EDTP is to react ethylene diamine and 1-chloro-2-propanol. This avoids the formation of higher hydroxypropylated derivatives of EDTP, but gives a product which contains not only the impurities present in ethylene diamine, but also those present in 1-chloro-2-propanol. In addition, 1-chloro-2-propanol is a very costly raw material compared to propylene oxide.

The commercially available EDTP is produced by one of the above described methods and is "impure" in the sense of the present invention. If distilled at 66 Pascal at a temperature of 184°C the fraction which distills over at 184°C is the pure EDTP, which has all the properties desired for its successful use in electroless plating baths, while the commercially available product is unacceptable for high quality electroless copper plating.

Surprisingly, it has been found that removal of small quantities of contaminants from the EDTP results in EDTP of uniform quality for electroless copper plating and allows the deposition of good quality copper. The contaminants removed are characterized in that they have lower boiling points compared to EDTP.

It has been further found that the removal of the low boiling contaminants must be done in such a way that no additional contaminants are formed as by-products of the removal process.

The distillation removes the low boiling contaminants as a fraction distilling below 184°C. Pure EDTP is distilled over at 184°C. The impurities produced by heating the EDTP are high boiling contaminants and are concentrated in the remaining undistilled fraction. The remaining undistilled fraction is no longer water white, but colored yellow.

This complete distillation of EDTP is expensive, and since only small quantities of low boilers are present, the preferred method is stripping off the low boiling impurities by distillation. Thus, stripping is best done at low temperatures in the absence of air or oxygen to avoid formation of oxidation by-products and other high boiling impurities. The stripping is done in an inert gas atmosphere or preferably under vacuum. Preferably, stripping to remove the low boiling contaminants should be carried out at very low pressures and low temperatures to avoid formation of high boilers as contaminants.

The aforementioned distillation procedures for obtaining the EDTP used herein must be carefully controlled in respect of the following parameters:

(a) rate of heating - if the impure or commercial EDTP is heated too fast, the solution becomes yellow or brown indicating formation of new impurities not initially present in the impure material;

(b) pressure control - higher pressures can be employed but, in such case, there is a tendency to produce decomposition and oxidation products during the distillation process. Therefore, 66 Pascal is most preferred because of the lower partial pressure of oxygen. The same applies to higher temperatures for vacuum stripping of EDTP; temperatures up to 50°C are preferred.

(c) vapor condensation - it should proceed at a steady state manner since any clogging during condensation will cause overheating resulting in decomposition of EDTP and an
occurrence of side reactions in the distillation
vessel.

It has been observed that copper deposited from an electroless copper bath in the absence of a brightener, which is
prepared with EDTP with the low boilers removed by vacuum
distillation up to 50°C abd 66 Pascal or EDTP which distills
over at the same pressure at 184°C has a brighter color, exhibits good physical properties and is not stressed.

In comparison, copper deposited from an impure EDTP containing bath is unacceptable: it is very dark and is stressed.
Although less preferred, other distillation techniques are
available to provide generally comparable EDTP as used herein. For instance, distillation pressures higher than 66 Pascal can be used and utilization of an inert gas, e.g., nitrogen during distillation as a stripping agent is a useful
variant.

Also applicable are procedures which are unlike the distillation procedures discussed above.

One such procedure involves adsorption chromatography in which
commercially available or impure EDTP can be purified by passing an aqueous solution of EDTP through a chromatographic
column which is packed with activated alumina, activated
carbon, silica or other high surface area adsorbents. The
chromatographic column is of the conventional design and flow
of substance to be purified can be accelerated by external
pressure applied by means of an inert gas. Fractions are
identified by and separated on the basis of color, refractive index and/or spectrally. EDTP for use as described herein can be obtained by this procedure.

Another method for producing EDTP for use herein is termed
pre-electrolysis. In this method, purification of impure or
commercial EDTP is obtained in the presence of copper sulfate
or other supporting electrolyte. During electrolysis, impurities are deposited or react at the electrodes and are
thus removed from the EDTP solution when the electrodes are
removed.

Another method for producing EDTP for use herein involves an ion exchange recycling process in which an EDTP solution is first pre-electrolysed (in an electrolytic or electroless bath) and then further purified with an adsorptive chromatographic step.

After EDTP is purified by one of the methods described hereinbefore, it should be handled in a manner to avoid decomposition and/or atmospheric oxidation. Otherwise, the EDTP may develop an unsuitable color characteristic of further contamination. It is found that heating purified EDTP above 95°C in air will induce coloration, i.e., the EDTP will turn yellow and, eventually, brown. Since yellowing is prevented when heating does not exceed 60°C, it is recommended that heating be kept below 60°C in handling.

Facile and easily followed methods have been established to ensure that the EDTP employed in electroless copper plating baths has the purity required to provide consistently good deposited products, i.e., the ability to consistently provide in a test solution a copper plating bath rate of between 4,8 and 8,8 µm per hour at a temperature of between 24 and 26°C and a copper deposit on a stainless steel strip (reference) which is bright in color, not stressed, and does not flake off due to stress cracking.

Such quality control methods include an electromechanical method, a functional test and a physio-chemical measurement. The electromechanical method for evaluating the purity of EDTP in a bath involves the cyclic voltametry of a formaldehyde-EDTP, a copper ion-EDTP or a complete electroless copper solution. The test electrode can be a stationary or a rotating electrode, polarized cathodically or anodically. The range of polarization is from -2,0 to +2,0 V vs. saturated calomel electrode (SCE).

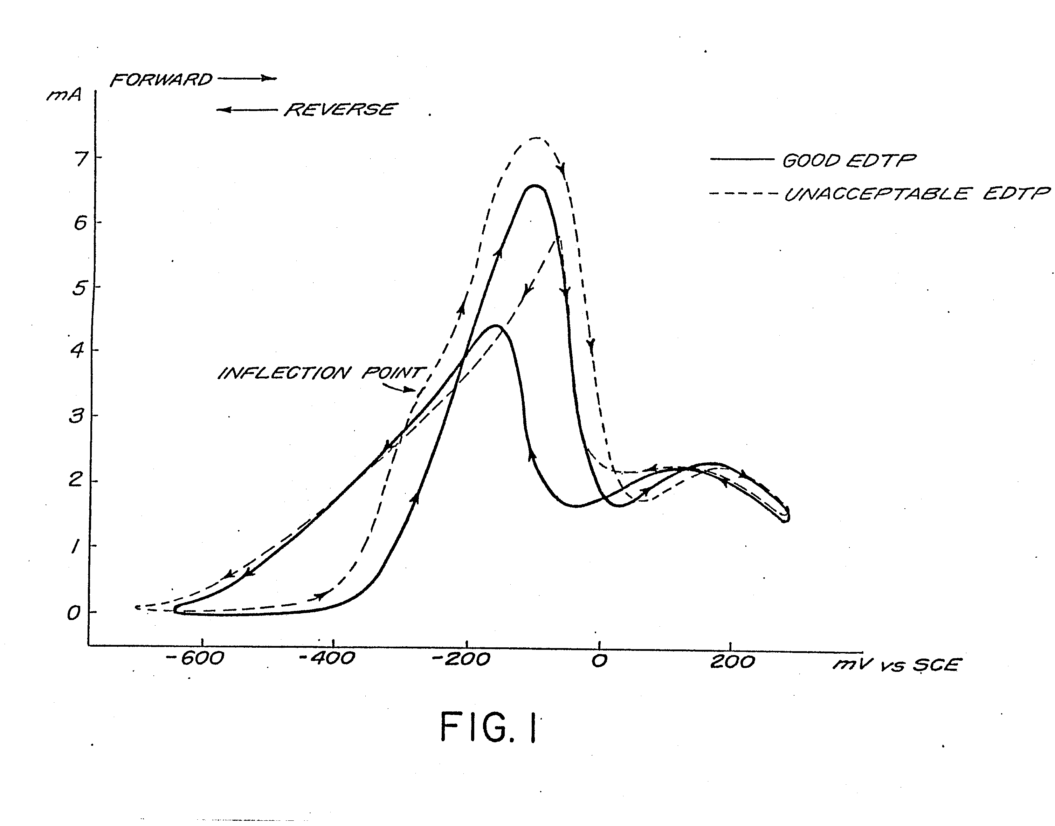

The Figure is a graphical presentation of the cyclic voltammograms of a platinum test electrode in a bath. There are shown two cyclic voltammograms for the anodic oxidation of formaldehyde in the presence of two commercial EDTP products. It can be seen that the voltammogram for the solution containing unacceptable EDTP shows an inflection point in the potential range between -400 and -200 mV vs. SCE.

The inflection point between -400 and -200 mV vs. SCE indicates the presence of an extraneous component which is being oxidized at that potential. The solution containing acceptable EDTP, as disclosed herein, does not show this inflection point. The peak current in the range between -200 and 0,0 mV vs. SCE is higher for the unacceptable than for the acceptable EDTP.

The higher peak current shows the extraneous component continues to be oxidized simultaneously with, or affects the oxidation of formaldehyde, since the peak current is mainly due to the oxidation of formaldehyde. This difference in the peak current is also indicative of the purity of EDTP. It is also noted that the position of the current peak (maximum) on the potential axis is different for two EDTP products.

In theory, since these extraneous materials are oxidizable at the same or a lower potential then formaldehyde, the said materials and their oxidation products can affect the copper deposition and may even be incorporated in the metal deposit.

A similar electrochemical test can be used to analyze EDTP in an electroless copper solution.

A functional quality control test for EDTP involves testing EDTP in an electroless copper bath having the following composition:

| | |
|---|---|
| EDTP | 35 g/l (0,12 Mol) |
| $CuSO_4 \cdot 5H_2O$ | 18 g/l (0,07 Mol) |
| $CH_2O$ (37%) | 20 ml/l |
| Sodium 2-mercaptobenzo-thiazole (MBT) | 1,5 mg/l |
| pH adjusted with NaOH | 13,3 |
| Temperature | 25 °C |

Copper-clad strips and stainless steel strips are activated for 30 seconds in 1 g/l $PdCl_2$ in 20 ml/l HCl and plated in the above shown composition for 15 to 30 minutes.

In the above procedure, when the copper bath contained a commercial EDTP, the rate of deposition was 9,4 μm per hour. The deposited copper was stressed and very dark.

However, when the EDTP was prepared in accordance with one of the methods described herein, the rate of deposition was between 4,8 and 8,8 μm per hour and the deposited copper had good physical properties, a bright color and was not stressed.

Particular advantages associated with this functional test are:

        (i) it is closest to the application of EDTP in the electroless plating bath;

      (ii) it does not require specialized equipment; and

     (iii) it does not require highly-trained personnel.

The physico-chemical method for evaluating the purity of EDTP can be achieved by one of several measurements, e.g., high-pressure liquid chromatography, boiling point determination, viscosity and by infrared (IR) adsorption spectrometry.

In high-pressure liquid chromatography measurements, the important quality citeria are the absence of high and particularly low molecular weight components. Impure EDTP when tested by high-pressure liquid chromatography has had as many as four major components. Purified EDTP has shown only two major components very close in molecular weight comprising 97 % of the product.

Since most of the impurities boil below 40°C at 66 Pascal, boiling point measurements monitor EDTP quality. Water is not a major component of these impurities since the impurities distilling over below 40°C at 66 Pascal do not condense in an ice trap, but condense in a liquid nitrogen trap.

The criteria for acceptable EDTP as used herein relate to rate and stress parameters. The plating rate at 24-26°C should fall between about 4,8 and 8,8 μm per hour. Regarding stressing, the deposit on the stainless steel strip must be smooth and there should be no evidence of flakey copper deposition. Smooth copper deposits on the stainless steel strip are unstressed. Deposits that have blisters or bubbles between copper and the stainless steel are slightly stressed. Severely stressed copper deposits stress-crack during deposition forming flakes or flakey copper. Flakey copper will fall off if the strip is tapped against a hard surface.

### EXAMPLE 1

An EDTP product (Quadrol[RTM]) (1 kg) was placed in a distillation flask and inserted into a conventional distillation apparatus connected to a vacuum pump. The EDTP was gently heated at a steady vapor condensation rate. Most of the impurities boiled off below 40°C at 66 Pascal. These impurities did not condense in an ice trap, but were condensed in a liquid nitrogen trap. When a distillation temperature of 184°C at 66 Pascal pressure was attained, the fraction which distills over was collected.

### EXAMPLE 2

Example 1 is repeated except that the distillation was stopped just at the point when the fraction at 184°C at 66 Pascal began to distill over. The residue was cooled and bottled.

### EXAMPLE 3

This Example provides a comparative demonstration of the use of EDTP as disclosed herein and commercially available EDTP (Quadrol[RTM]) in a room temperature electroless copper deposition bath having the following composition:

| | |
|---|---|
| Copper sulfate pentahydrate | 18 g/l |
| Formaldehyde (37% solution) | 20 ml/l |
| Sodium 2-mercaptobenzothiazole | 1,5 mg/l |
| Sodium hydroxide | 36,9 g/l |
| pH | 13,3 |
| Temperature | 25 - 27°C |

To the room temperature bath was added 17,5 g/l of the following EDTP products: EDTP (Example 1); EDTP (Example 2); and Quadrol[RTM]. For each formulation, the following test was performed: copper-clad strips and stainless steel strips were activated for 30 sec. in 1 g/l PdCl$_2$ in 20 ml/l HCl and plated in the above plating bath formulation (1 liter test baths) for 15 minutes to observe the incidence of stressing.

The copper foil test strips were used to determine the plating rate. The following results were obtained:

| Properties | EDTP (Ex.1) | stripped at non-preferred temperatures EDTP (Ex.2) | Quadrol[RTM] 'as received' EDTP (Ex.3) |
|---|---|---|---|
| Rate (μm/hour) | 5,49 | 7,70 | 10,43 |
| Color & brightness | pink | tan | dark brown |
| Stress | smooth non-stressed | slight stress bubbles | stressed, flaked |
| Solution stability | stable | stable | stable |

The EDTP of Example 1 shows excellent results. The EDTP of Example 3 shows completely unacceptable results. The EDTP of Example 2 shows improved but not satisfactory results due to the formation of by-products forming at the higher destillation temperature which were not separated from the EDTP.

EXAMPLE 4

A lot of commercial EDTP which had been selected as the most satisfactory for electroless copper plating out of all lots available in the manufacturer's warehouse was stripped of low boiling impurities at a temperature of 40°C at 66 Pascal. The low boiling impurities obtained by stripping were 1,5 ml per liter of EDTP.

Plating solutions were prepared as in Example 3 using EDTP as received and EDTP after stripping of low boiling impurities. The following results were obtained:

| Properties | As received | After stripping |
|---|---|---|
| Plating rate (μm/h) | 8,1 | 6,9 |
| Color | pink | pink |
| Stress | fair, small bubbles | good, smooth, no bubbles |

This test shows that even the best of commercially available EDTP has high plating rates, indicative of poor plating bath stability and stress in the deposits. Removal of the low boiling impurities resulted in an improved plating bath with plating rates indicative of long term stability and in unstressed deposits.

### EXAMPLE 5

This Example provides a comparative demonstration of the use of EDTP as disclosed herein and commercially available EDTP in hot electroless copper plating baths used for the manufacture of fully-additive printed circuit boards.

In the manufacture of fully-additive printed circuit boards a 35 to 40 µm thick copper deposit obtained by electroless deposition forms all the conductors and plated through holes on a circuit board. When all the copper on a circuit board is electrolessly deposited, the electroless copper bath must deposit a ductile copper layer to insure the integrity of the copper conductors and plated through holes. It is prior art to form suitable copper deposits by inclusion of ductility promotors such as cyanide and the like in the electroless plating bath solution.

Cyanide and other ductility promotors greatly improve the ductility of the deposits from electroless copper plating solutions using EDTP as the complexing agent. Even impure EDTP solutions may deposit somewhat ductile copper but greatly improved ductility is obtained with pure EDTP of this invention as is shown by plating from solutions of the following composition:

#### Hot Bath Composition

| | | |
|---|---|---|
| EDTP | 17,5 | g/l |
| Copper sulfate pentahydrate | 10,5 | g/l |
| Gafac RE 610[RTM] * | 0,16 | g/l |
| Formaldehyde (37% solution) | 3,5 | ml/l |
| Sodium hydroxide | 8,0 | g/l |
| Sodium cyanide | 30,0 | mg/l |

Potassium polysulfide

  (sulfurated potash)          0,6  mg/l

Sodium 2-mercaptobenzothiazole  0,075 mg/l

pH  (at 25°C)               12,7

Temperature              53°C

This hot electroless bath is used in the manufacture of fully additive printed circuit boards. A 35 to 40 µm thick copper deposit obtained from this composition forms all the conductors and plated through holes on a circuit board.

*Nonylphenylpolyglycidolphosphate ester surfactant


The hot electroless plating bath was run in a 360 liter plating tank for 4 days producing 4 lots of printed circuit boards. An examination of plating rate, color, stress, ductility and solution stability is performed using stainless steel strips and actual additive circuit board test panels. All samples are plated for 16 to 18 hours.

The copper deposited from the solution containing impure commercial EDTP had marginal ductility while the copper deposited from the solution containing only pure EDTP in accordance with this invention had excellent ductility. The ductility is measured by bending the copper deposit through 180°, in one direction, creasing, then returning it to its original position, with pressing along the crease to flatten it, this cycle constituting one bend. Non-ductile copper deposits break below 1 1/2 bends; low ductility copper deposits break between 1 1/2 and 3 bends; and high ductility copper deposits break at a higher number of bends.

The copper deposited from solutions containing impure EDTP broke over a range of 1 to 2 bends, while copper deposited from solutions containing purified EDTP broke over a range of 4 to 6 bends.


## EXAMPLE 6

A commercially available EDTP is divided into two parts; one part is used as received while the second part is stripped at 50°C and 500 Pascal to produce a purified EDTP free of low boilers. A fraction less than 1% by weight is removed by stripping from this second part. This fraction does not condense as within an ice trap, but is a low boiling organic contaminant which only condenses in a liquid nitrogen trap.

Two electroless copper plating solutions were prepared as in Example 3 using the two different EDTP parts. The copper deposited from the solution containing the EDTP as received was dark brown and stressed; the copper deposited from the solution containing the stripped EDTP had a pinkish copper color and the deposit was very slight stressed.


## EXAMPLE 7

A column is packed with activated alumina. It is flushed with deionized water to displace all air. A solution of 50% impure EDTP in deionized water is prepared. The EDTP is forced through the column by pressurized nitrogen gas. The deionized water used to flush air from the column exits first and is discarded. A dilute EDTP solution exits next and is also discarded. A clear solution of EDTP in water solution changes color, all the purified EDTP solution has been collected. A plating solution is prepared as in Example 3. The copper deposited has a pink color and is not stressed.


## EXAMPLE 8

A solution is prepared with

| Impure EDTP | 350 | g |
| Copper sulfate | 170 | g |
| Water to make | 1 | liter |

A copper anode and a copper cathode are placed in the solution and a current is passed between the anode and the cathode sufficient to maintain a current density of 10 mA/cm$^2$. After pre-electrolysis over night, the solution is cleansed of impurities.

An electroless copper plating solution is prepared as in Example 3. Equivalent results are obtained.

CLAIMS:

1. An electroless copper bath for the deposition of copper onto a substrate comprising at least a water soluble copper salt, formaldehyde, hydroxide to adjust the pH, a wetting agent and ethylenedinitrilotetra-2-propanol (EDTP) as complexer for the copper ions, characterized in that the low boiling impurities contained in commercially available EDTP are removed by a process during which no oxidation by-products are formed.

2. The electroless copper bath of claim 1 characterized in that the EDTP complexer does not contain more than 0,2% of low boiling impurities.

3. The electroless copper bath of claim 1 characterized in that the low boiling impurities are removed by chromatographic methods.

4. The electroless copper bath of claim 1 characterized in that the low boiling impurities are stripped.

5. The electroless copper bath of claim 1 characterized in that the low boiling impurities are removed by electrolytic methods.

6. The method of claim 4 characterized in that the stripping is done by vacuum destillation.

7. The method of claim 6 characterized in that the destillation is done under reduced pressure between 65 and 1500 Pascal.

8. The method of claim 6 characterized in that the destillation is done at below 100 Pascal.

9. The method of claims 6 to 8 characterized in that the low boiling impurities are removed by destillation under reduced pressure and at temperatures below 150°C.

10.     The method of claims 6 to 9 characterized in that the low boiling impurities are removed at a temperature of below 50°C, preferably at 40°C.

11.     The method of claim 6 characterized in that the destillation is done in N₂ atmosphere.

12.     An electroless plating bath in accordance with claim 1 comprising 18 g/l of copper sulfate pentahydrate, 20 ml/l formaldehyde (37%), 1,5 mg/l sodium-2-mercapto-benzothiazole and 37 g/l sodium hydroxide, characterized in that it further contains 18 g/l of EDTP containing substantially no low boiling impurities.

13.     The electroless plating bath of claim 12 characterized in that it has a plating rate on a reference copper foil of between 4,8 and 8,8 μm per hour at a temperature of between 24° and 26°C, and that the copper deposited on said reference strip is smooth, non-flakey, unstressed and bright in color.

14.     The electroless plating bath of claim 12 characterized in that it consistently provides plating rates of between 4,8 and 8,8 μm per hour at a temperature of between 24° and 26°C and a copper deposit which is smooth, non-flakey, unstressed and bright in color.

FIG. 1

1/1

0116283

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | CHEMICAL ABSTRACTS, vol. 91, 1979, page 305, no. 171004n, Columbus, Ohio, USA G. FRANK: "A cheap and simple method to achieve and maintain the necessary purity of reagents and solvents for automated amino acid sequence determination with the sequenator" & HOPPE-SEYLER'S Z. PHYSIOL. CHEM. 1979, 360(7), 997-999 * Abstract * | | C 23 C 3/00 C 07 C 91/00 |
| | --- | | |
| A | CHEMICAL ABSTRACTS, vol. 94, 1981, page 304, no. 152798v, Columbus, Ohio, USA A.S. BHOWN et al.: "An improved procedure for high-sensitivity microsequencing: use of aminoethyl aminopropyl glass beads in the Beckman sequencer and the ultrasphere ODS column for PTH amino acid identification" & ANAL. BIOCHEM. 1981, 110(2), 355-359 * Abstract * | | |
| | --- | | **TECHNICAL FIELDS SEARCHED (Int. Cl. ³)** C 23 C C 07 C |
| A,D | US-A-3 119 709 (ATKINSON) | | |
| | --- | | |
| A,D | US-A-2 697 118 (LUNDSTED) | | |
| | ----- | | |

The present search report has been drawn up for all claims

| Place of search THE HAGUE | Date of completion of the search 03-05-1984 | Examiner NGUYEN THE NGHIEP |
|---|---|---|